# EUROPEAN PATENT SPECIFICATION

(11) **EP 1 363 651 B1**
(45) Date of publication and mention of the grant of the patent: **14.09.2005**
(21) Application number: 02711728.2
(22) Date of filing: 26.02.2002
(51) Int. Cl.: A61K 35/78

(54) **HERBAL COMPOSITIONS USEFUL AS CHEMOPREVENTIVE AND THERAPEUTIC AGENTS**
KRÄUTERPFLANZEN ENTHALTENDE ARTZNEIMITTEL
COMPOSITIONS A BASE D'HERBES MEDICINALES UTILISEES COMME AGENTS CHIMIO-PREVENTIFS ET THERAPEUTIQUES

(30) Priority: 26.02.2001 CA 793251
(43) Date of publication of application: 26.11.2003
(73) Proprietor: Global Cancer Strategies Ltd., British Columbia V6E 4M2 (CA)
(72) Inventor: TZE, John, Wah Jun, (CA); LIN, Peizhong, Cancer Inst. Chi. Aca. of Med. Sci., Beijing 100021 (CN); LAM, Stephen, Vancouver, British Columbia V6T 2E3 (CA); TAI, Joseph, North Vancouver, British Columbia V7L 1T (CA)
(74) Representative: van Westenbrugge, Andries
(86) International application number: PCT/CA2002/000204
(87) International publication number: WO 2002/067960

(56) References cited:
- EP-A- 0 568 001
- EP-A- 0 665 017
- GB-A- 2 226 494
- US-A- 4 459 285

## Description

The present invention relates to compositions comprising herbs and their use as chemopreventive and therapeutic agents.

### BACKGROUND OF THE INVENTION

For the past twenty-five years there has been significant progress in the field of cancer research; however, in spite of these positive results, the mortality rate for the most common cancers still remains high. Indeed, the goal of the National Cancer Institute of a fifty percent reduction in overall cancer mortality by the year 2000 has not been met.

The term "cancer" is a general one referring to more than 100 forms of the disease which may manifest itself in almost every tissue type of the body. Of the myriad forms of cancer, lung cancer is the most common cause of death worldwide, followed by stomach cancer. Other common forms of cancer include cancers of the colon, rectum, breast, prostate, mouth and esophagus.

Lung cancer imposes an enormous burden on health care. The World Health Report 2000 estimates that lung cancer has resulted in 860,000 deaths among men and 333,000 deaths among women per year, and it is the leading cause of cancer deaths worldwide. In the United States and Canada, more people are dying from lung cancer than from breast cancer, colorectal cancer and prostate cancer combined. In addition, the incidence of lung cancer in women is rising at an average annual rate of 7% which is the most rapid rate of increase for any cancer. The most dominant cause of lung cancer is tobacco use, but occupational and environmental exposure to various other carcinogenic substances can also influence disease development. In long-term smokers, the risk of lung cancer never returns to the "baseline" level of a never-smoker, even years after smoking cessation. With a large reservoir (100 million in the United States and Canada alone) of current and former smokers, who are at risk, lung cancer will continue to be a major health problem for at least several more decades even if current efforts to curb tobacco smoking were successful. The overall five-year survival rate of lung cancer is less than 15%. Despite advances in modern medicine, the survival rate has not improved substantially over the last two decades. A different approach is therefore needed to control lung cancer.

Prostate cancer is the most commonly diagnosed male malignancy in the United States and the second leading cause of cancer death. It is estimated that in the year 2000, there will be 180,400 new cases and 31,900 deaths caused by prostate cancer. Although prostate cancer responds effectively to orchitectomy or antiandrogen therapy when detected at an early stage, over time, the residual androgen-insensitive cells recolonize, expand, and ultimately establish a hormone-resistant state that often results in fatality. It would be useful, therefore, to have a cancer treatment which could prevent the proliferation of prostate cancer and maintain it in a dormant state.

The incidence of gastric cancer has fallen in most countries but it is still the most common form of cancer in many countries of East Asia, including China. Globally, gastric cancer is the second most frequent cause of cancer death and it is estimated that in 1990, there were almost 800,000 new cases and about 630,000 deaths. Similarly, esophageal cancer, the eighth most common cancer worldwide and responsible for 316,000 new cases and 286,000 deaths in 1990, is also very common in China and other Asian countries. Both of these cancers, and especially esophageal cancer, have low survival rates and thus it would be beneficial to have an alternative treatment approach for these types of cancers.

Traditionally, the focus of cancer research has been in developing therapies and treatments for patients already afflicted with the disease. However, over the last few decades, new insights into the development of cancer as a disease have been gained. It is now understood that cancer is not the result of a single initiation event but of a gradual, multi-step process characterized by a period of several years between the initiation event and the onset of invasive or metastatic disease. In general, the process of carcinogenesis can be divided into three phases: initiation, promotion, and progression. In initiation, a fixed genetic mutation results from the interaction of a carcinogen with DNA. The extent of the molecular change depends on a number of factors including the nature of the carcinogen, the rate and type of carcinogenic metabolism and the response of the DNA repair systems. The next phase, promotion, may occur over extended periods of time and is characterized by the proliferation of the altered cells. This phase may be affected by agents that alter growth rates. During the final phase, progression, genetic and phenotypic changes occur which ultimately cause the development of premalignant lesions into invasive cancer.

The multi-step nature of carcinogenesis suggests the possibility of intervention at a precancerous state. This is the basis for chemoprevention, which refers to the use of natural or synthetic agents to prevent the development of cancer, either by blocking the DNA damage that initiates the carcinogenesis process or by arresting/regressing existing pre-malignant lesions.

Since the mid-1950s, research has been directed at finding compounds with potential chemopreventive properties. The search for these agents has demonstrated a unique challenge. Chemopreventive agents must have low toxicity and be relatively free of side effects because they are intended for administration to healthy people over long periods of time. This is in direct contrast to chemotherapy drugs, such as cis platinum or paclitaxel (Taxol™), which are used as chemotherapeutic agents to treat people already afflicted with cancer. Chemotherapeutic agents are chosen for their ability to kill tumor cells but because they are also toxic to healthy cells, they usually cause harmful side effects.

One of the major sources of potential chemopreventive agents is plants. For example, consumption of cruciferous vegetables, such as broccoli, cauliflower and cabbage is associated with a lower risk of various cancers. Fruits and vegetables contain a number of potentially active chemopreventive compounds, such as carotenoids, dithiolthiones and isothiocyanates. They are capable of inhibiting the development of tumors of the lungs, colon, mammary glands and bladder in laboratory animals.

Three proof-of-principle clinical trials suggest that chemoprevention might be an effective strategy to control lung cancer. A study by Hong and co-workers in the United States showed that in patients with cured head and neck cancer, high dose 13-cis-retinoic acid for 12 months was more effective than placebo in preventing second primary cancers in the upper aerodigestive tract. However, 13-cis-retinoic acid at this dosage carries unacceptable toxicity for use in the general population. Another study by Pastorino and co-workers in Europe showed that retinol palmitate in a dose of 300,000 Units per day for 12 months was more effective than placebo in preventing second primary lung cancer. A third study in China found that daily doses of a combination of vitamins and minerals consisting of beta-carotene, vitamin E and selenium resulted in a 21 % decrease in stomach cancer deaths in high-risk people in China. However, subsequent phase III clinical trials using beta-carotene or retinal (the Alpha-Tocopherol, Beta-Carotene Trial, the Beta-Carotene and Retinol Efficacy Trial, and the EUROSCAN study) failed to show a reduction in lung cancer incidence in high-risk individuals, such as heavy smokers with or without exposure to asbestos, compared to placebo. In fact, the use of beta-carotene in those who continued to smoke during the study was found to increase the risk of lung cancer. Several reasons were postulated to explain why chemopreventive treatment with retinoids was ineffective or even harmful in active smokers. There may be adverse interactions between tobacco carcinogens and the chemopreventive agent. Beta-carotene, for example, is a pro-oxidant at high arterial oxygen tension. It can enhance conversion of benzo[A]pyrene to the ultimate carcinogen as well as inducing cytochrome P450. Another reason for the lack of effect in active smokers is that ongoing tobacco carcinogen exposure may counteract the effect of the chemopreventive agent.

A number of chemopreventive agents are currently under clinical investigation. Examples of these include fenretinide, selenium, inhaled budesonide, COX-2 inhibitors, farnesyl transferase inhibitors, lipoxygenase inhibitors, EGF-kinase inhibitors and green tea. Although promising, it remains to be shown if these agents can be shown to be useful in ongoing clinical trials.

The best example to-date that chemoprevention can prevent cancer is Tamoxifen. Tamoxifen is an estrogen antagonist. In women at high risk of breast cancer, the Breast Cancer Prevention Trial showed a 49% decrease in invasive cancer and a 50% decrease in non-invasive breast cancer with Tamoxifen versus placebo (J Natl Cancer Inst 1998; 90:1371-88). In addition, there was a decrease in the incidence of fractures due to osteoporosis. However, there was a slight increase in the risk of endometrial cancer, deep venous thrombosis and pulmonary embolism. More selective estrogen-receptor modulators such as Raloxifene, are being tested against Tamoxifen to determine if these agents may have similar chemopreventive effect but fewer side effects than Tamoxifen.

A variety of Chinese herbs have been used for centuries to treat different diseases. The great majority of these are empirical, open, non-randomized studies without placebo control groups. Although some of these herbs have been used to treat patients with cancer, they are not considered to be disease specific. Rather, they are used for "dispersing heat, detoxification, improving stasis and removing mass". Herbs such as *Sophora tonkinensis, Polygonum bistorta, Prunella vulgaris, Sonchus brachyotus, Dictamnus dasycarpus Turcz,* and *Dioscorea bulbifera* are known to have properties that may be useful for the prevention and treatment of cancers. One such composition is known as ZSP or Zeng Sheng Ping; however, the exact formulation of the composition is not known.

European Patent application 93 121109.8 describes a composition comprising *Sophora tonkinensis* Gapnep 42 mg, *Polygonum bistorta* L. 42 mg, *Prunella vulgaris* L. 42 mg, *Sonchus brachyotus* DC 42 mg, *Dictamnus dasycarpus Turcz* 21 mg, and *Dioscorea bulbifera* 10 mg which was reported to be useful for the treatment of patients with mouth, esophagus or digestive tract cancers. Thus, there is a need to develop other herbal remedies for the treatment and prophylaxis of common cancers, and for other therapeutic uses.

### SUMMARY OF THE INVENTION

Thus, according to the present invention there is provided a composition comprising herbs.

Thus, according to this embodiment of the invention, there is provided a composition comprising a mixture of at least three different herbs selected from the group consisting of herbs, which for the purpose of this invention have been designated as: Herb A, Herb B, Herb C, Herb D, Herb E and Herb F.

In the composition defined in EPO 93121109.8 the herb corresponding to Herb A was *Sophora tonkinensis (Sophora subprostrata)*. According to the present invention it has been found that any one of the following herbs can be used in place of *Sophora tonkinensis*:
*Belamcanda chinensis*
*Scrophularia ningpoensis.*
*Isatis tinctoria*
*Isatis indigotica* or
*Baphicacanthus cusia Bremek.*

In the composition defined in EPO 93121109.8 the herb corresponding to Herb B was *Polygonum bistorta*. According to the present invention it has been found that any one of the following herbs can be used in place of *Polygonum bistorta*:
*Polygonum lapidosum*
*Polygonum viviparum*
*Polygonum manshuriense*
*Polygonum alopecuroides*
*Polygonum sphaerostachyum*
*Andrographis paniculata*
*Taraxacum mongolicum* or
*Chrysanthemun indicum*.

In the composition defined in EPO 93121109.8 the herb corresponding to Herb C was *Prunella vulgaris*. According to the present invention it has been found that any one of the following herbs can be used in place of *Prunella vulgaris:*
*Artemissia capillaris*
*Gardenia jasminoides*
*Rosa rugosa* or
*Lophatherum gracile.*

In the composition defined in EPO 93121109.8 the herb corresponding to Herb D was *Sonchus brachyotus*. According to the present invention it has been found that any one of the following herbs can be used in place of *Sonchus brachyotus:*
*Patrinia scabiosaefolia*
*Patrinia villosa*
*Sonchus arvensis*
*Thlaspi arvense*
*Portulaca oleracea* or
*Pulsatilla chinensis.*

In the composition defined in EPO 93121109.8 the herb corresponding to Herb E was *Dictamnus dasycarpus*. According to the present invention it has been found that any one of the following herbs can be used in place of *Dictamnus dasycarpus:*
*Kochia scoparia*
*Sophora flavescens* or
*Heydyotis diffusa*.

In the composition defined in EPO 93121109.8 the herb corresponding to Herb F was *Dioscorea bulbifera*. According to the present invention it has been found that any one of the following herbs can be used in place of *Dioscorea bulbifera*:
*Panax notoginseng*
*Bletilla striata*
*Nelumbo nucifera*
*Cephalanoplos segetum*
*Cirsium japonicum*
*Typha angustifolia*. or
*Rubia cordifolia*.

Further according to the present invention there is provided a use of said composition as chemopreventive and therapeutic agents.

### DETAILED DESCRIPTION OF PREFERRED EMBODIMENT

The present invention relates to compositions comprising herbs and their use as chemopreventive and therapeutic agents.

In one embodiment of the present invention the composition comprises a mixture of at least three different herbs, known hereinafter as Herb A, Herb B, Herb C, Herb D, Herb E and Herb F.

According to the present invention Herb A is selected from the group consisting of:
*Belamcanda chinensis*
*Scrophularia ningpoensis*
*Isatis tinctoria*
*Isatis indigotica* and
*Baphicacanthus cusia*.

According to the present invention Herb B is selected from the group consisting of:
*Polygonum lapidosum*
*Polygonum viviparum*
*Polygonum manshuriense*
*Polygonum alopecuroides*
*Polygonum sphaerostachyum.*
*Andrographis paniculata*
*Taraxacum mongolicum* and
*Chrysanthemun indicum.*

According to the present invention Herb C is selected from the group consisting of:
*Artemissia capillaris*
*Gardenia jasminoides*
*Rosa rugosa* and
*Lophatherum gracile.*

According to the present invention Herb D is selected from the group consisting of:
*Patrinia scabiosaefolia*
*Patrinia villosa*
*Sonchus arvensis*
*Thlaspi arvense*
*Portulaca oleracea* and
*Pulsatilla chinensis.*

According to the present invention Herb E is selected from the group consisting of:
*Kochia scoparia*
*Sophora flavescens* and
*Heydyotis diffusa.*

According to the present invention Herb F is selected from the group consisting of :
*Panax notoginseng.*
*Bletilla striata*
*Nelumbo nucifera*.
*Cephalanoplos segetum .*
*Cirsium japonicum*.
*Typha angustifolia* and
*Rubia cordifolia*.

When, according to the present invention, the composition comprises three different herbs, one of which is selected from each of Herb A, Herb B, Herb C, Herb D or Herb E, each of the three herbs are present in an amount from 9% to 57%. In a further example of this embodiment, the three herbs are each present in an amount from 15 % to 50%. In a further example of this embodiment, each of the three herbs are present in an amount from 25 % to 40%. In a further example of this embodiment, each of the three herbs are present in an amount of 33%.

When the composition comprises four different herbs, one of which is selected from each of Herb A, Herb B, Herb C, Herb D or Herb E, each of the four herbs are present in an amount from 6 % to 43%. In a further example of this embodiment, each of the four herbs are present in an amount from 15 % to 35 %. In a further example of this embodiment, each of the four herbs are present in an amount of 25 %.

In a further example of the present invention, when the composition comprises four herbs, three of which are selected from the group consisting of Herb A, Herb B, Herb C, Herb D and the fourth herb is selected from the group consisting of Herb E. The composition of the first three herbs are each present in an amount from 7 % to 57% and the fourth herb is present in an amount from 3.5 % to 28.5%. In a further example of this embodiment, each of the first three herbs are present in an amount from 15% to 48% and the fourth herb is present in an amount from 7.5% to 24%. In a further example of this embodiment, the first three herbs are present in a composition in an amount of 20 % to 43%, and the fourth herb is present in an amount from 10% to 21.5%. In a further example of this embodiment, the first three herbs are present in an amount of 28% and the fourth herb is present in an amount of 14%.

In yet a further embodiment of this invention, the composition comprises five different herbs, one of which is each selected from Herb A, Herb B, Herb C, Herb D or Herb E, each of the five herbs are present in an amount from 5% to 35 %. In a further example of this embodiment, each of the five herbs are present in an amount from 10% to 30%. In yet a further example of this embodiment, each of the five herbs are present in an amount from 15 % to 25 %. In a further example of this embodiment, each of the five herbs are present in an amount of 20%.

In yet a further embodiment of this invention, the first four herbs are selected one from each of the group Herb A, Herb B, Herb C and Herb D whereas the fifth herb is selected from Herb E and the first four herbs are present in an amount from 6% to 38%, whereas the fifth herb is present at an amount from 3 % to 19%. In a further example of this embodiment, the first four herbs are present in the composition at an amount from 12 % to 32 %, and the fifth herb is present in an amount from 6% to 16%. In yet a further example of this embodiment, the first four herbs are present in an amount from 18 % to 26% and the fifth herb is present in an amount from 9% to 13%. In yet a further example of this embodiment, the first four herbs are present at an amount of 22% , whereas the fifth herb is present at an amount of 11 %.

In yet a further embodiment, the composition comprises six herbs. The first five herbs are selected from the group consisting of Herb A, Herb B, Herb C, Herb D and Herb E, and the sixth herb is selected from Herb F and the first five herbs are present in an amount from 5% to 31 % and the sixth herb is present in an amount from 2.5 % to 17.5%. In a further example of this embodiment, each of the first five herbs are present in an amount from 10% to 26 % , and the sixth herb is present in an amount from 5% to 15 %. In a further example of this embodiment, the first five herbs are each present in an amount from 15 % to 21% and the sixth herb is present in an amount from 7.5 to 12.5 %. In a further example of this embodiment, the first five herbs are present in an amount from 18% and the sixth herb is present in an amount from 10%.

In yet a further example of this embodiment, the first four herbs are selected from the group of Herb A, Herb B, Herb C or Herb D. The fifth herb is selected from Herb E and the sixth herb is selected from Herb F, wherein the first three herbs are each present in an amount from 5% to 38%, the fifth herb is present in an amount from 2.5 % to 19 % and the sixth herb is present in an amount from 1.75 % to 9.5%. In a further example of this embodiment, the first four herbs are present in an amount from 10% to 33%. The fifth herb is present in an amount from 5% to 16.5% and the sixth herb is present in an amount from 3% to 8.25%. In yet a further example of this embodiment, the first four herbs are present in an amount from 15 % to 28%; herb five is present in an amount from 7.5% to 14.5% and the sixth herb is present in an amount from 4.25% to 7%. In yet a further example of this embodiment, the first four herbs are present in an amount from 21%. The fifth herb is present in an amount from 10.5 % and the sixth herb is present in the amount from 5.5%.

It is important to ensure that the herbs which are used according to the present invention are selected such that they contain only acceptable levels of contaminants such as metals or pesticides. Various regions of China have been surveyed and it was found that the component herbs can be harvested from the Guangxi, Hunan, Liaoning, Anhui, Hebei and Jiangsu regions of China during the summer and autumn months and no pesticides are used. The plants are purchased dried and whole or parts of these herbs are used in manufacturing.

The general manufacturing scheme is as follows: the herbs are either subjected to an aqueous extraction, the aqueous extract is then filtered if necessary to remove large particles, the aqueous extract is then dried to a powder. Alternatively, it is possible to use the herbs directly by grinding to a powder. The powdered herbs are then used in the production of the therapeutic in a variety of forms for administration.

Any suitable mode of delivery can be used according to the present invention. For example the composition of the present invention can be delivered orally by a pill, tablet, drink, candy or paste. The composition can also be delivered as a transdermal patch, by inhalation or suppository. Delivery of the composition as an injectable is also possible, according to the present invention. Therefore, the composition can be administered as a therapeutic agent or as a dietary supplement.

In one embodiment of the present invention, tablets are formulated into 0.3g tablets. A typical daily dosage is 1.2 to 6 g/day based on the body weight and/or severity of the condition.

According to the present invention the compositions are useful for a variety of therapeutic treatments. In one embodiment of the present invention the compositions are useful for the prevention or treatment of cancers.

According to one embodiment of the present invention, the composition comprises additional therapeutic agents. Examples of such agents can include chemotherapeutic agents.

## Claims

1. A composition comprising a mixture of at least three different herbs selected from the group consisting of, : Herb A, Herb B, Herb C, Herb D, Herb E and Herb F; wherein
Herb A is selected from the group consisting of:
*Belamcanda chinensis*
*Scrophularia ningpoensis.*
*Isatis tinctoria*
*Isatis indigotica* and
*Baphicacanthus cusia;*
Herb B is selected from the group consisting of
*Polygonum lapidosum*.
*Polygonum viviparum*
*Potygonum manshuriense*
*Polygonum alopecuroides*
*Polygonum sphaerostachyum*
*Andrographis paniculata*
*Taraxacum mongolicum* and
*Chrysanthemun indicum;*
Herb C is selected from the group consisting of:
*Artemissia capillaris*
*Gardenia jasminoides*
*Rosa rugosa* and
*Lophatherum gracile;*
Herb D is selected from the group consisting of:
*Patrinia scabiosaefolia*
*Patrinia villosa*
*Sonchus arvensis*
*Thlaspi arvense*
*Portulaca oleracea* and
*Pulsatilla chinensis;*
Herb E is selected from the group consisting of:
*Kochia scoparia*
*Sophora flavescens and*
*Heydyotis diffusa;* and
Herb F is selected from the group consisting of:
*Panax notoginseng*
*Bletilla striats*
*Nelumbo nucifers*
*Cephalanoplos segetum*
*Cirsium japonicum*.
*Typha angustfolia and*
*Rubia cordifolia*

2. The composition of claim 1, wherein the composition comprises three herbs, one of which is selected from each of Herb A, Herb B, Herb C, Herb D or Herb E and wherein each is present in an amount from 9% to 57%.

3. The composition of claim 1, wherein the composition comprises four herbs, one of which is selected from each of Herb A, Herb B, Herb C, Herb D or Herb E, and wherein each is present in an amount from 6% to 43%.

4. The composition of claim 1, wherein the composition comprises four herbs, the first three are selected from the group consisting of Herb A, Herb B, Herb C and Herb D and are present in an amount from 7% to 57% and the fourth is selected from Herb E and is present in an amount from 3.5% to 28.5%.

## Patentansprüche

1. Zusammensetzung, aufweisend eine Mischung von mindestens drei verschiedenen Kräuterpflanzen, ausgewählt aus der Gruppe von Kräuterpflanze A, Kräuterpflanze B, Kräuterpflanze C, Kräuterpflanze D, Kräuterpflanze E und Kräuterpflanze F; wobei
- Kräuterpflanze A ausgewählt ist aus der Gruppe von:
*Belamcanda chinensis,*
*Scrophularia ningpoensis*,
*Isatis tinctoria*,
*Isatis indigotica* und
*Baphicacanthus cusia*;
- Kräuterpflanze B ausgewählt ist aus der Gruppe von:
*Polygonum lapidosum*,
*Polygonum viviparum*,
*Polygonum manshuriense*,
*Polygonum alopecuroides*,
*Polygonum sphaerostachyum*,
*Andrographis paniculata*,
*Taraxacum mongolicum* und
*Chrysanthemun indicum;*
- Kräuterpflanze C ausgewählt ist aus der Gruppe von:
*Artemissia capillaris*,
*Gardenia jasminoides*,
*Rosa rugosa* und
*Lophatherum gracile;*
- Kräuterpflanze D ausgewählt ist aus der Gruppe von:
*Patrinia scabiosaefolia*,
*Patrinia villosa*,
*Sonchus arvensis*,
*Thlaspi arvense,*
*Portulaca oleracea* und
*Pulsatilla chinensis;*
- Kräuterpflanze E ausgewählt ist aus der Gruppe von:
*Kochia scoparia*,
*Sophora flavescens* und
*Heydyotis diffusa*; und
- Kräuterpflanze F ausgewählt ist aus der Gruppe von:
*Panax notoginseng,*
*Bletilla striats*,
*Nelumbo nucifers,*
*Cephalanoplos segetum,*
*Cirsium japonicum*,
*Typha angustfolia* und
*Rubia cordifolia*.

2. Zusammensetzung nach Anspruch 1, wobei die Zusammensetzung drei Kräuterpflanzen aufweist, von denen eine ausgewählt ist aus jeweils Kräuterpflanze A, Kräuterpflanze B, Kräuterpflanze C, Kräuterpflanze D oder Kräuterpflanze E, und wobei jede in einer Menge von 9 % bis 57 % vorhanden ist.

3. Zusammensetzung nach Anspruch 1, wobei die Zusammensetzung vier Kräuterpflanzen aufweist, von denen eine ausgewählt ist aus jeweils Kräuterpflanze A, Kräuterpflanze B, Kräuterpflanze C, Kräuterpflanze D oder Kräuterpflanze E, und wobei jede in einer Menge von 6 % bis 43 % vorhanden ist.

4. Zusammensetzung nach Anspruch 1, wobei die Zusammensetzung vier Kräuterpflanzen aufweist, wobei die ersten drei ausgewählt sind aus der Gruppe von Kräuterpflanze A, Kräuterpflanze B, Kräuterpflanze C und Kräuterpflanze D und in einer Menge von 7 % bis 57 % vorhanden sind und wobei die vierte ausgewählt ist aus Kräuterpflanze E und in einer Menge von 3,5 % bis 28,5 % vorhanden ist.

## Revendications

1. Composition comprenant un mélange d'au moins trois herbes différentes choisies parmi une Herbe A, une Herbe B, une Herbe C, une Herbe D, une Herbe E et une Herbe F, dans laquelle
L'herbe A est choisie parmi :
*Belamcanda chinensis*
*Scrophularia ningpoensis*
*Isatis tinctoria*
*Isatis indigotica* et
*Baphicacanthus cusia;*
L'herbe B est choisie parmi :
*Polygonum lapidosum*
*Polygonum viviparum*
*Polygonum manshuriense*
*Polygonum alopecuroides*
*Polygonum sphaerostachyum*
*Andrographis paniculata*
*Taraxacum mongolicum* et
*Chrysanthemum indicum ;*
L'herbe C est choisie parmi :
*Artemissia capillaris*
*Gardenia jasminoides*
*Rosa rugosa* et
*Lophatherum gracile ;*
L'herbe D est choisie parmi :
*Patrinia scabiosaefolia*
*Patrinia villosa*
*Sonchus arvensis*
*Thlasni arvense*
*Portulaca oleracea* et
*Pulsatilla chinensis;*
L'herbe E est choisie parmi :
*Kochia scoparia*
*Sophora flavescens* et
*Heydyotis diffusa ;* et
L'herbe F est choisie parmi :
*Panax notoginseng*
*Bletilla striats*
*Nelumbo nucifers*
*Cephalanoplos segetum*
*Cirsium japonicum*
*Typha angustfolia* et
*Rubia cordifolia*

2. Composition selon la revendication 1, dans laquelle la composition comprend trois herbes médicinales dont l'une est choisie parmi chacune de l'herbe A, l'herbe B, l'herbe C, l'herbe D ou l'herbe E et dans laquelle chacune est présente dans une quantité de 9% à 57%.

3. Composition selon la revendication 1, dans laquelle la composition comprend quatre herbes médicinales dont l'une est choisie parmi chacune de l'herbe A, l'herbe B, l'herbe C, l'herbe D ou l'herbe E, et dans laquelle chacune est présente dans une quantité de 6% à 43%.

4. Composition selon la revendication 1, dans laquelle la composition comprend quatre herbes médicinales, les trois premières sont choisies parmi l'herbe A, l'herbe B, l'herbe C et l'hérbe D et sont présentes dans une quantité de 7% à 57% et la quatrième est choisie à partir de l'herbe E et est présente dans une quantité de 3,5% à 28,5%.
